# EUROPEAN PATENT SPECIFICATION

(11) **EP 0 439 993 B1**
(45) Date of publication and mention of the grant of the patent: **06.03.1996**
(21) Application number: 90630262.5
(22) Date of filing: 21.12.1990
(51) Int. Cl.: A61B 17/56, A61F 2/00

(54) **Apparatus for gauging and controlling process steps used to remove prosthethic joints**
Vorrichtung zum Abmessen und Steuern von Verfahrensschritten zur Entfernung künstlicher Gelenke
Appareil pour jauger et contrôler des pas d'un procédé pour écarter des articulations prothétiques

(30) Priority: 19.01.1990 US 467742; 19.01.1990 US 467724; 06.02.1990 US 475778
(43) Date of publication of application: 07.08.1991
(73) Proprietor: ZIMMER, INC., Warsaw, Indiana 46580 (US)
(72) Inventor: Chin, Albert K., Palo Alto, California 94306 (US); McColl, Milton B., Los Altos Hills, California 94022 (US); Watkins, F. Thomas, Menlo Park, California 94025 (US)
(74) Representative: Mays, Julie

(56) References cited:
- WO-A-90/03769
- CH-A- 566 127

## Description

### Related Patent

This application relates to U.S. Patent 4,919,153 in the name of Albert K. Chin, one of the co-inventors herein.

### Background of the Invention

U.S. Patent 4,919,153 is concerned with a method and apparatus for removing the cement mantle used to secure a prosthetic appliance within a bone cavity and, more particularly, is concerned with a technique wherein a new mass of cement is adhered in place within the mantle and a pulling tool is then threaded into the mass and employed to remove the mass and the mantle as a unit. Swiss patent CH-A-566 127 is concerned with a pulling tool for removing a cement plug from the intramedullary canal. The tool comprises a cylindrical shaft with a cutting screw at the distal end and has a proximal T-shaped handgrip. PCT patent application No. WO 90/03769 cited under A54(3) and (4) EPC for the commonly designated states concerns a method and apparatus for removing a pre-placed prosthesis from a cement cavity. A pulling appliance is inserted into fluid cement placed within the cement cavity. The fluid cement is permitted to cure to bond the appliance to the mantel. The pulling appliance is of screw-like configuration with means to connect a slap-hammer to its proximal end. In the present invention, the cement mantle and newly injected mass of cement are removed in increments to avoid the severe stresses which are sometimes encountered in removing the cement mantle and newly injected mass of cement as a single unit. The invention is particularly concerned with an arrangement to avoid overtightening of the pulling tool and resulting fracture of the cement being removed.

### Summary of the Invention

The present invention employs a sleeve telescopically received on the pulling tool. The sleeve has an inner diameter providing a sliding fit with the tool and an outer diameter small enough to allow it to pass down the femoral canal. An indicator or stop ring is provided on the pulling tool to the proximal end of the sleeve. In use, the sleeve is initially disposed so as to expose only a limited number of threads at the distal portion of the tool. The tool is introduced into the threaded plug of new cement and threaded into place. As the tool is threaded into the plug, the sleeve is displaced proximally toward the indicator or stop ring. When the sleeve reaches the indicator or ring, the surgeon knows that the tool has been threaded into place to the proper depth.

The present invention also comprises a gauge to measure the depth of the screw-threaded passage formed in the plug of cement. The gauge enables the surgeon to determine how many successive tools will be required to affect the incremental removal of the entire plug. The gauge also provides means to measure the number of turns to which the last of the successive tools may be turned into place. This number is generally less than the total number of turns for which the tool is designed, since the last segment of the plug is rarely the exact length of the threads provided on the pulling too.

The gauge takes the form of an elongate rod having a diameter less than that of the threaded passage being measured. A tube having a diameter greater than that of the passage is slidably disposed around the rod. Indicia on the rod indicate the longitudinal position of the tube relative to the rod.

A principal object of the invention is to provide an improved apparatus to control the extent to which pulling tools are threaded into a cement plug being used to remove the cement mantle for a prosthetic joint.

Another and more specific object of the invention is to provide such an apparatus which avoids overtightening of the pulling tool and stripping of the threads within the plug.

A further and more general object of the invention is to provide a simplified and essentially foolproof apparatus for avoiding overtightening the pulling tools used for incrementally removing a cement mantle from a bone cavity.

These and other objects will become more apparent when viewed in light of the following detailed description and accompanying drawings.

### Brief Description of the Drawings

Fig. 1 is an elevational view, with parts thereof shown in section, of the kit of basic elements used to practice the invention, including the thread forming die, four successive pulling tools, and the gauge;
Fig. 2 is a cross-sectional elevational view of a modified form of a pulling tool which may be used in the practice of the invention;
Fig. 3 is a cross-sectional elevational view of the upper femur of a leg, wherein the femoral component of a prosthetic hip joint has been removed from a cement mantle within the femur and a new mass of cement is in the process of being injected into the cavity left in the mantle by removal of the component;
Fig. 4 is a cross-sectional elevational view similar to Fig. 3, illustrating the step of using a thread forming die to form a threaded passage within the mass of new cement injected into the mantle;
Fig. 5 is a cross-sectional elevational view similar to Fig. 4, illustrating removal of the thread forming die from the mass of new cement to leave a threaded passage therein;
Fig. 6 is cross-sectional elevational view similar to Fig. 5, illustrating the use of the gauge to measure the depth of the threaded passage in the mass of new cement.
Fig. 7 is a cross-sectional elevational view similar to Fig. 6, illustrating the first of the successive pulling tools at the commencement of being threaded into the passage within the plug comprised of the mass of new cement and the cement of the old mantle;
Fig. 8 is a cross-sectional elevational view similar to Fig. 7, illustrating the first of the successive pulling tools at the termination of being threaded into the passage within the plug;
Fig. 9 is a cross-sectional elevational view similar to Fig. 8, illustrating a slap hammer coupled to the first pulling tool and in the process of breaking away and removing a segment of the plug;
Fig. 10 is a cross-sectional elevational view similar to Fig. 9, illustrating the plug of cement which remains after the step of Fig. 9;
Fig. 11 is a cross-sectional elevational view similar to Fig. 10, illustrating the second of the successive pulling tools at the commencement of being threaded into the passage within the remaining plug of cement;
Fig. 12 is a cross-sectional elevational view similar to Fig. 11, illustrating the second of the successive pulling tools at the termination of being threaded into the passage within the remaining plug of cement;
Fig. 13 is a cross-sectional elevational view similar to Fig. 12, illustrating a slap hammer connected to the second pulling tool and in the process of breaking away and removing a second segment of the plug of cement;
Fig. 14 is a cross-sectional elevational view similar to Fig. 13, illustrating the gauge in the process of measuring the depth of the threaded passage in the plug of cement remaining after the step of Fig. 13;
Fig. 15 is a cross-sectional elevational view similar to Fig. 14, illustrating the third of the successive pulling tools at the commencement of being threaded into the passage within the remaining plug of cement;
Fig. 16 is a cross-sectional elevational view similar to Fig. 15, illustrating the third of the successive pulling tools at the termination of being threaded into the passage within the remaining plug of cement;
Fig. 17 is a cross-sectional elevational view similar to Fig. 16, illustrating a slap hammer coupled to the third pulling tool and in the process of breaking away and removing a segment of the plug of cement;
Fig. 18 is a cross-sectional elevational view similar to Fig. 17, illustrating the fourth of the successive pulling tools at the commencement of being threaded into the passage within the remaining plug of cement;
Fig. 19 is a cross-sectional elevational view similar to Fig. 18, illustrating the fourth of the successive pulling tools at the termination of being threaded into the passage within the remaining plug of cement; and,
Fig. 20 is a cross-sectional elevational view similar to Fig. 19, illustrating the fourth pulling tool coupled to a slap hammer and in the process of breaking away and removing the final segment of the plug of cement.

### Description of the Preferred Embodiments

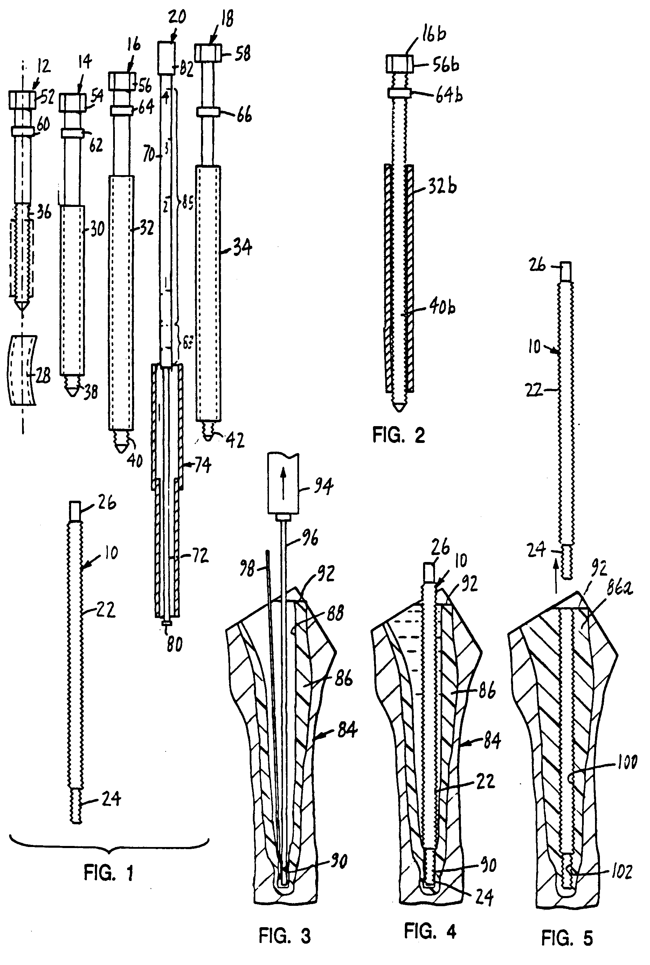

The kit shown in Fig. 1 includes the following components: thread forming die **10,** first successive pulling tool **12,** second successive pulling tool **14,** third successive pulling tool **16,** fourth successive pulling tool **18,** and depth gauge **20.** The die **10** includes a main portion **22** of a uniform enlarged cross-section and a distal end portion **24** of a reduced cross-section, as compared to that of the main portion. Both the main portion **22** and the distal portion **20** are threaded over their lengths with continuous screw threads of the same pitch (e.g. machine screw pitch 20). The top end of the die is provided with a square **26** whereby the die may be turned with a wrench. The die may be formed of a metal or a polymer and ideally is provided with a non-stick external surface over the threaded portions. The non-stick surface may be a permanent integral part of the die, such as TEFLON (registered trade mark), and/or a release coating, such as DOW CORNING 20 of the Dow Corning Corporation of Midland, Michigan.

The pulling tools **12, 14, 16** and **18** are each of a bolt-like construction and fabricated of steel rod, with a polymer sleeve telescopically received thereon. The sleeves for the tools **12**, **14**, **16** and **18** are designated, respectively, by the numerals **28**, **30**, **32** and **34**. In a typical embodiment, the tool **12** has a length of 10.16 cm (four inches), the tool **14** 15.24 cm (six inches), the tool **16** 20.32 cm (eight inches) and the tools **18** 22.86 cm (nine inches). Each tool terminates in a conical distal end and has a distal portion adjacent said end formed with external machine screw threads. The threaded sections formed on the tools **12**, **14** and **16** are designated by the numerals **36**, **38** and **40**, respectively, and have a length of approximately 3,81 cm (1-1/2 inches) and an o.d. of 0,63 cm (1/4 inch). The threaded section of the tool **18** has a length of approximately 1,9 cm (3/4 of an inch) and an o.d. of 0,32 cm (1/8 inch). The screw threads on the pulling tools are complemental with the threads formed by the die **10**.

The tools **12**, **14**, **16** and **18** are formed with unthreaded sections above the threaded sections thereof. Enlarged hexagonal heads **52**, **54**, **56** and **58**, respectively, are formed on the proximal ends of the tools **12**, **14**, **16** and **18**. The heads provide means whereby torsional and pulling forces may be applied to the tools.

Stop collars **60, 62**, **64** and **66** are fixed, respectively, to the tools **12**, **14**, **16** and **18**. The collars serve as markers and abutments for the sleeves **28**, **30**, **32** and **34**. The sleeves are proportioned to frictionally engage the pulling tools to an extent sufficient to resist inadvertent sliding movement relative thereto, while permitting such movement in response to external forces applied to the sleeves as they are threaded into a plug of cement. This proportioning may be provided by cutting the sleeves from an arcuate length of tubing, whereby each sleeve has an arcuate shape (see the sleeve **28** in Fig. 1) which is straightened as the sleeve is telescoped onto a pulling tool. Alternatively, it might be provided by forming the sleeves of tubing having an internal diameter slightly less than the external diameter of the threaded sections of the tools and slitting the sleeves longitudinally to permit their expansion. The lengths of the sleeves **28, 30, 32** and **34** are chosen so that, when engaged with the stop collars, the sleeves will cover only the most proximal screw threads of the tools. As a result of the latter dimensional interrelationship, a surgeon using one of the tools will visually observe the sleeve on the tool approaching the stop collar as the tool is threaded into place. When the sleeve contacts the stop collar, the surgeon will know that the tool is fully threaded into place and that no additional torsional forces should be applied to the tool. It will be appreciated that when fully threaded into place, the sleeves on the tools function to shield the most proximal threads of the tools against being threaded into place in the cement being engaged. Thus, the tool cannot be threaded against the shoulder provided by the unthreaded portion thereof.

Fig. 2 shows a modified pulling tool which corresponds to the tool **16,** except that the shaft of the tool is threaded over its full length, rather than a limited distal portion. The elements of the tool shown in Fig. 2 are designated by numerals corresponding to those used for the tool **16,** followed by the subscript "b", as follows: tool **16**_{**b**}**;** sleeve **32**_{**b**}**;** threaded section **40**_{**b**}**;** hexagonal head **56**_{**b**}**;** and stop collar **64**_{**b**}**.** In use, the tool **16**_{**b**} operates in the same manner as the aforedescribed tools. Although the full length of the shaft of the tool **16**_{**b**} is externally threaded, the sleeve **32**_{**b**} serves to shield the threads on all but the distal portion of the shaft. The tool **16**_{**b**} has the advantage that it may be fabricated of fully threaded rod stock, without the necessity of providing an unthreaded section on the tool shaft.

The gauge **20** is provided to measure the depth of the screw threaded passage formed by the die **10.** The elements of the gauge comprise: an indicator rod **68** having an enlarged diameter proximal portion **70** and a reduced diameter distal portion 72; a tube 74 telescopically received on the rod 68, said tube having an enlarged segment proportioned for slidable receipt of the proximal portion 70 and a reduced diameter segment 78 proportioned for slidable receipt of the distal portion 72; an enlarged tip 80 fixed to the end of the distal portion 70. The external surface of the proximal portion 70 is provided with indicia to indicate the longitudinal position of the rod 68 relative to the tube 74. The indicia at the lower end of the proximal portion 70 are designed by the numeral 83 and calibrated to measure screw thread turns. The indicia on the upper length of the proximal portion 70 are designated by the numeral 85 and calibrated to measure the number of pulling tools required to fully remove the mantle of cement being worked upon.

The use of the apparatus is depicted sequentially in Figs. 3 to 20. As there shown, the femur being worked upon is designated in its entirety by the numeral 84 and is illustrating after the femoral component of a prosthetic hip joint has been removed therefrom for replacement. These figures also show that the trochanter of the femur has been removed to facilitate the method.

Fig. 3 shows the femur **84** after the femoral component of the hip joint has been removed, with the cement mantle **86** which is to be removed left in place within the bone recess within the femur. As there shown, the cavity **88** within the mantle has been cleaned and a reduced diameter extension, designated **90,** has been drilled into the distal end of the mantle. The top of the mantle has also been cut to provide a horizontal surface, as designated by the numeral **92.**

The first step of preparing the mantle for removal comprises injecting cement into the cavity **88.** This step is illustrated in Fig. 3 wherein an injection gun **94** is shown injecting cement to the bottom of the cavity through a thin snout **96.** A vent tube **98** is extended to the bottom of the cavity to assure that air will be vented therefrom and that the cavity will be filled to the bottom. As the cavity is filled with cement, the gun and snout are slowly retracted, as depicted by the arrow line in Fig. 3. The vent tube **98** would be withdrawn after the reduced diameter extension **90** is adequately filled. Most typically, the mantle **86** is comprised of old methylmethacrylate cement. This type of cement is capable of being partially dissolved and softened by the application of new like fluid cement thereto. Accordingly, assuming that the mantle is comprised of such cement, the new cement injected into the cavity **88** would be a like cement and, ultimately, bond to the original mantle and form an integral part thereof.

Fig. 4 shows the mantle filled with new cement to the level of the horizontal surface **92** and the step of forming a screw threaded passage through the plug of new cement. As there shown, the die **10** has been screwed to essentially the bottom of the cavity to form a screw threaded passage therein. It should also be appreciated that the die is provided with a non-stick coating prior to being so screwed into place, either in the form of an integral surface formed as part of the die and/or a non-stick coating applied to the die.

Fig. 5 shows the mantle, now in the form of a unitary plug **86**_{**a**}**,** after the newly injected cement has cured and formed an integral mass with the mantle. As shown in Fig. 5, the die **10** has been threaded out of the plug, leaving a passage comprised of an enlarged proximal portion **100** and a reduced diameter distal portion **102.**

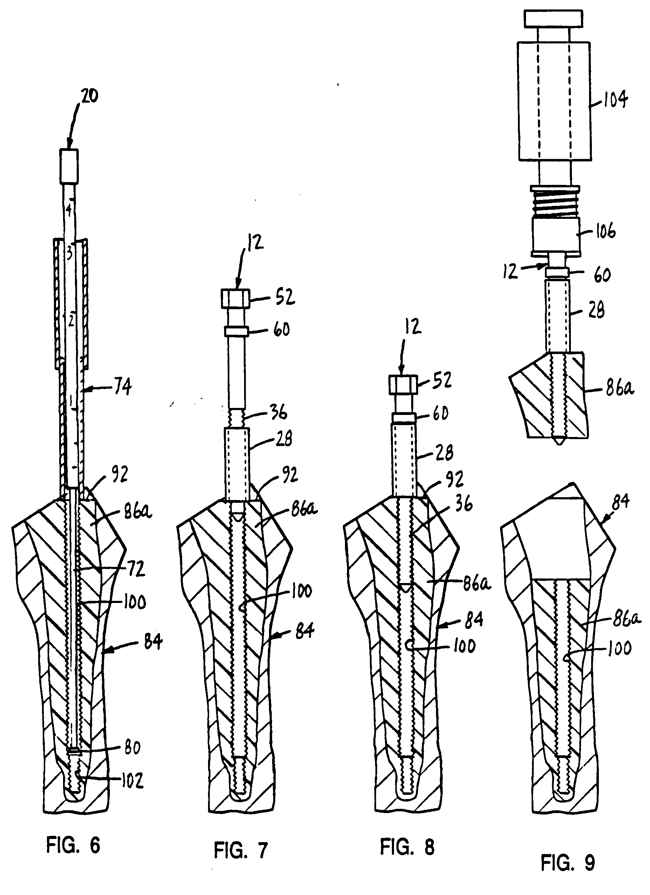

Fig. 6 shows the gauge **20** in the process of measuring the depth of the passage within the plug **86**_{**a**}**.** As there shown, it will be seen that the rod **68** is extended to the bottom of the proximal portion **100** of the passage. This figure also illustrates that the rod has a diameter less than the internal diameter of the passage and that the tip **80** has a cross-section less than the portion **100** and greater than the portion **102.** As a result of the latter characteristic, the tip engages the shoulder between the portions **100** and **102** and the depth being measured by the gauge is actually that of only the portion **100.** From Fig. 6, it will be seen that the upper edge of the tube **74** registers with a portion of the indicia **85** between the numerals 3 and 4 indicating the number of tools which will be required to remove the mantle. This means that four tools will be required for the process and that the third tool will need to be threaded into less than the full length of the threaded section on the tool.

Figs. 7 and 8 show the first tool **12** in the process of being threaded into place in the mantle plug **86**_{**a**}**.** As shown in Fig. 7, the distal threads only of the tool are engaged and the sleeve **28** is engaged with the surface **92.** Fig. 8 shows the tool **12** threadably engaged with the proximal portion **100** of the passage to the maximum extent desired, as indicated by contact of the sleeve **28** with the stop **60.** It will be appreciated that in the process of being threaded from the condition shown in Fig. 7 to that shown in Fig. 8, the surgeon would visually observe the sleeve **28** sliding toward the stop **60** and stop the threading process when the pulling tool reaches the point shown in Fig. 8.

Fig. 9 shows the tool **12** connected to a slap hammer **104** through a coupling **106.** As there shown, pulling force has been applied to the upper portion of the mantle plug **86**_{**a**} and that portion or segment, designated **86**_{**a1**} has been removed from the femur **84.** Such removal is possible because a methylmethacrylate cement has very little tensile strength and readily fractures upon being subjected to tensile force by the slap hammer.

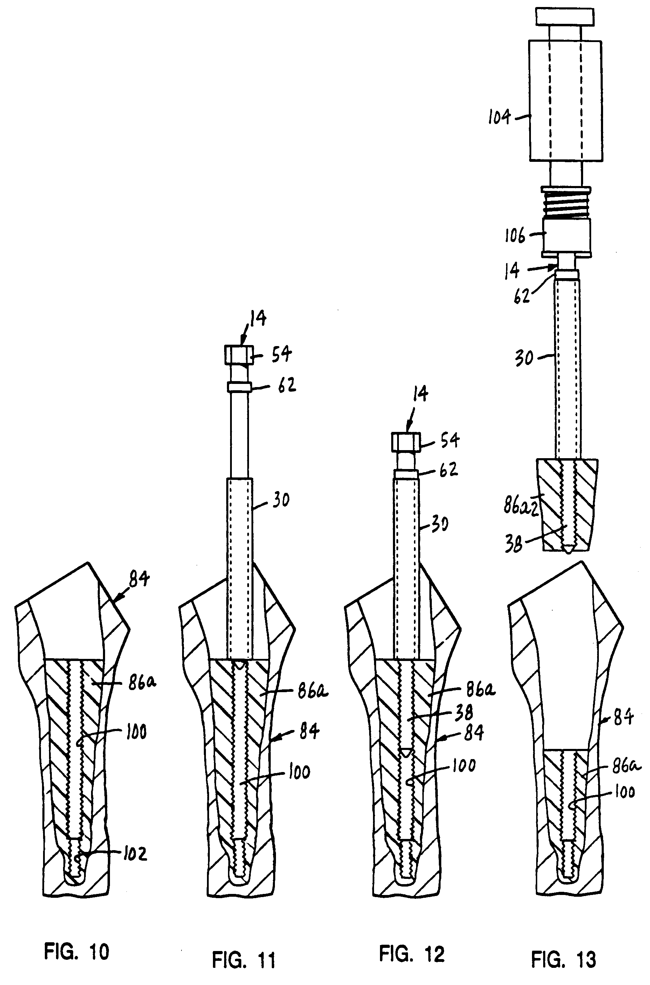

Figs. 11 and 12 show the second pulling tool **14** in the process of being threaded into place in the mantle plug **86**_{**a**} in essentially the same manner that the first pulling tool is depicted as being threaded into place in Fig. 7 and 8. In Fig. 11, the threading process is at its commencement, with the sleeve **30** against the top surface of the mantle plug. Fig. 12 shows the tool **14** threaded into the mantle plug to the full extent desired, as indicated by abutment of the sleeve **30** with the stop **62.**

Fig. 13 shows the slap hammer **104** connected to the tool **14** and the segment **86**_{**a2**} as having been removed from the femur by the operation of the slap hammer. Again, it should be appreciated that the low tensile strength of the methylmethacrylate cement enables the segment **86**_{**a2**} to be fractured away from the portion of the mantle plug remaining in the femur.

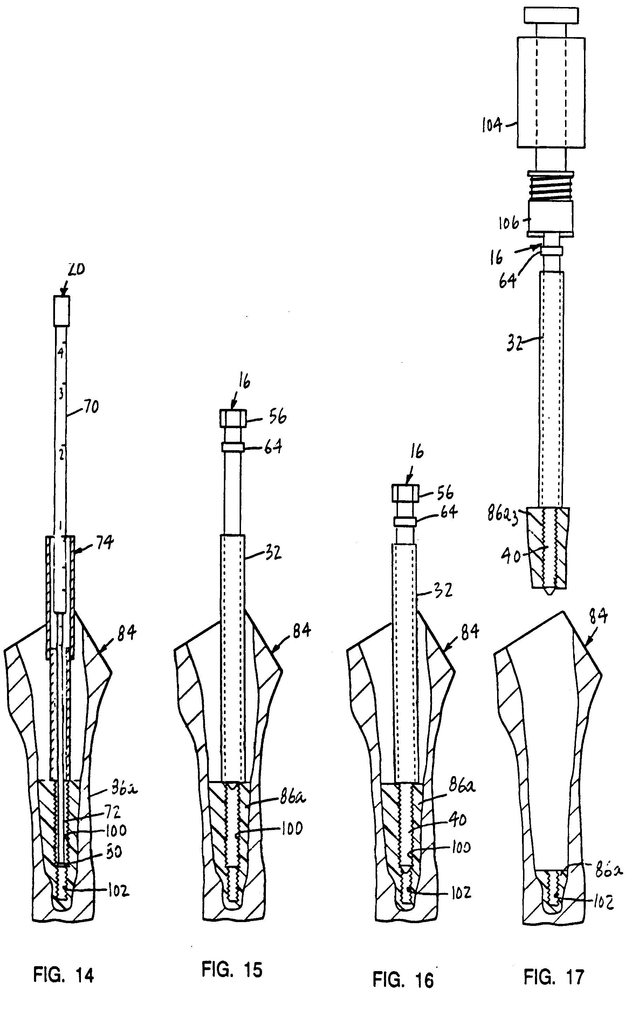

Fig. 14 shows the gauge **20** in the process of measuring the length of the proximal passage portion 100 remaining after removal of the segment **86**_{**a2**}**.** From this figure, it will be seen that the upper end of the tube **74** registers with the screw thread measuring indicia **83** on the rod **68.** This results because the depth of the threads being measured is less than the maximum exposed threads on a pulling tool when the sleeve on the tool is against the stop collar. The resulting measurement on the rod indicates the number of turns required to thread a pulling tool to the bottom of the threaded proximal portion **100,** without overtightening.

Figs. 15 and 16 illustrate the third pulling tool **16** in the process of being threaded into the remaining cement mantle plug **86**_{**a**}**.** As shown in Fig. 15, the threading process is at the commencement stage and the sleeve **32** is against the top of the plug. Fig. 16 shows the tool threaded into the plug to the maximum extent and illustrates that the sleeve **32** has not abutted the stop collar **64.** The latter condition results because the tool has been threaded into place by the number of turns indicated by the gauge in the step depicted in Fig. 14; and this number of turns results in less than full extension of the threaded section **40** from the sleeve **32.**

Fig. 17 shows the slap hammer **104** connected to the tool **16** and a segment **86**_{**a3**} as having been removed from the femur by operation of the slap hammer. As there shown, it will be seen that only the distal portion of the mantle plug remains within the femur.

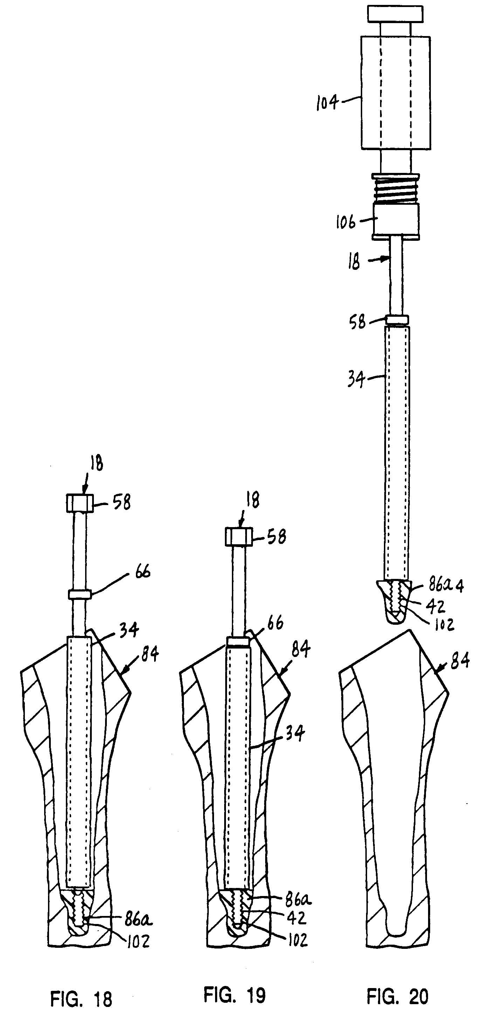

Figs. 18 and 19 depict the step of threading the fourth tool **18** into the final reduced diameter segment of the mantle. As shown in Fig. 18, the process of threading the tool into the mantle segment is at its commencement. Fig. 19 shows the tool fully threaded into place, as is apparent from contact of the sleeve **34** with the stop **66.** It will be appreciated that during the process moving from the condition shown in Fig. 18 to that of Fig. 19, the surgeon visually observes the sleeve **34** moving toward the stop collar **66** and exercises care not to tighten the tool beyond the point where the sleeve contacts the collar.

Fig. 20 shows the final step of removing the mantle segment 86ₐ₄ from the femoral cavity. As there shown, the slap hammer 104 is connected to the tool 18 and the plug segment 86ₐ₄ has been removed by operation of the slap hammer.

### Conclusion

From the foregoing description, it should be apparent that the present invention provides an apparatus to avoid the overtightening of the tools used for the incremental removal of cement mantles for prosthetic joints. At the same time, the invention provides a means whereby the surgeon may be fully informed in advance of the length of the mantle being removed and the number of steps which will be required for its removal.

While preferred embodiments of the invention have been illustrated and described, it should be understood that the invention is not intended to be limited to the specifics of these embodiments, but rather is defined by the accompanying claims.

## Claims (Claims for the following Contracting State(s): AT, BE, CH, DE, FR, GB, IT, LI, NL, SE)

1. A kit for removing a mantle of cement having an elongate cavity formed therein from adhered condition within a bone recess, said kit comprising a pulling tool (12) consisting of an elongate element (12) having a screw threaded section, said pulling tool (12) further comprising:
(a) a sleeve (28) slidably received on the elongate element, said sleeve (28) having a length less than that of the element; and,
(b) marker means (60) on the elongate element to enable a comparison to be made of the position of the sleeve (28) relative to the element, said marker means (60) comprising a stop (60) on the elongate element (12) disposed for abutment with the sleeve (28).

2. A kit as claimed in any preceding claim wherein the elongate element (12) comprises:
(a) a first rigid cylindrical post having proximal and distal ends, and having an exterior surface of a helical screw thread formed around said post; and,
(b) a second rigid cylindrical post attached to the distal end of said first post, said second post being reduced diameter as compared to said first post and extending axially therefrom.

3. A kit as claimed in claim 2 wherein the pulling tool further comprises means (106) on said proximal section for securing a slap hammer to said first post.

4. A kit as claimed in claim 2 or claim 3 wherein the second post has an exterior surface of a second helical screw thread formed around said second post.

5. A kit as claimed in any preceding claim wherein the kit further comprises:
(a) a die (10) for forming an elongate screw threaded passage within a mass of cement injected into the cavity;
(b) a second elongate element (14) of a predetermined length greater than that of the first element (12), said second element (14) having a screw threaded section (38) complemental with a screw threaded passage formed by the die;
(c) a second sleeve (30) slidably received on the second elongate element (14), said sleeve having a length less than that of the second element (14); and,
(d) marker means (62) on the second elongate element (14) to enable a comparison to be made of the position of the second sleeve (30) relative to the second element;
(e) torque transmitting means (52, 54) on said first and second elements to enable said elements to be screwed into threaded engagement with a threaded passage formed in a mass of cement by the die; and
(f) grippable means (106) on said first and second elements to enable pulling forces to be applied thereto to remove a mass of cement threadably engaged by the screw threaded sections (36,38) of the elements (12,14) from a bone recess; and in that the first elongate element 12) has a screw threaded section (36) complemental with a screw threaded passage formed by the die (10).

6. A kit as claimed in claim 5 wherein the kit further comprises a gauge (20) for measuring the length of the screw threaded passage.

7. A kit as claimed in claim 6 wherein the gauge (20) comprises:
(a) an elongate rod (68) having a diameter less than that of the screw threaded passage and a length greater than that of said passage;
(b) a tube (74) slidably disposed around the rod, said tube having a distal portion of a cross section greater than the diameter of the threaded passage; and,
(c) indicia means (85) operatively associated with the rod (68) and tube (74) to indicate the longitudinal position of the tube relative to the rod.

8. A kit as claimed in any preceding claim wherein the marker means comprises a collar fixed to the elongate element for abutment with the sleeve.

## Claims (Claims for the following Contracting State(s): ES)

1. A kit for removing a mantle of cement having an elongate cavity formed therein from adhered condition within a bone recess, said kit comprising a pulling tool (12) consisting of an elongate element (12) having a screw threaded section, characterised in that the pulling tool (12) further comprises:
(a) a sleeve (28) slidably received on the elongate element, said sleeve (28) having a length less than that of the element; and,
(b) marker means (60) on the elongate element to enable a comparison to be made of the position of the sleeve (28) relative to the element.

2. A kit as claimed in any preceding claim characterised in that the marker means (60) comprises a stop (60) on the elongate element disposed for abutment with the sleeve (28).

3. A kit as claimed in any preceding claim characterised in that the elongate element (12) comprises:
(a) a first rigid cylindrical post having proximal and distal ends, and having an exterior surface of a helical screw thread formed around said post; and,
(b) a second rigid cylindrical post attached to the distal end of said first post, said second post being of reduced diameter as compared to said first post and extending axially therefrom.

4. A kit as claimed in claim 3 characterised in that the pulling tool further comprises means (106) on said proximal section for securing a slap hammer to said first post.

5. A kit as claimed in claim 3 or claim 4 characterised in that the second post has an exterior surface of a second helical screw thread formed around said second post.

6. A kit as claimed in any preceding claim characterised in that the kit further comprises:
(a) a die (10) for forming an elongate screw threaded passage within a mass of cement injected into the cavity;
(b) a second elongate element (14) of a predetermined length greater than that of the first element (12), said second element (14) having a screw threaded section (38) complemental with a screw threaded passage formed by the die;
(c) a second sleeve (30) slidably received on the second elongate element (14), said sleeve having a length less than that of the second element (14); and,
(d) marker means (62) on the second elongate element (14) to enable a comparison to be made of the position of the second sleeve (30) relative to the second element;
(e) torque transmitting means (52, 54) on said first and second elements to enable said elements to be screwed into threaded engagement with a threaded passage formed in a mass of cement by the die; and
(f) grippable means (106) on said first and second elements to enable pulling forces to be applied thereto to remove a mass of cement threadably engaged by the screw threaded sections (36,38) of the elements (12,14) from a bone recess; and in that the first elongate element 12) has a screw threaded section (36) complemental with a screw threaded passage formed by the die (10).

7. A kit as claimed in claim 6 characterised in that the kit further comprises a gauge (20) for measuring the length of the screw threaded passage.

8. A kit as claimed in claim 7 characterised in that the gauge (20) comprises:
(a) an elongate rod (68) having a diameter less than that of the screw threaded passage and a length greater than that of said passage;
(b) a tube (74) slidably disposed around the rod, said tube having a distal portion of a cross section greater than the diameter of the threaded passage; and,
(c) indicia means (85) operatively associated with the rod (68) and tube (74) to indicate the longitudinal position of the tube relative to the rod.

9. A kit as claimed in any preceding claim wherein the marker means comprises a collar fixed to the elongate element for abutment with the sleeve.

## Patentansprüche (Patentansprüche für folgende(n) Vertragsstaat(en): AT, BE, CH, DE, FR, GB, IT, LI, NL, SE)

1. Vorrichtung zum Entfernen eines in einem haftenden Zustand in einer Knochenvertiefung angeordneten Zementmantels mit einem darin ausgebildeten länglichen Hohlraum, wobei die Vorrichtung ein Ziehwerkzeug (12) aufweist, das aus einem länglichen Element (12) mit einem Gewindeabschnitt gebildet wird, wobei das Ziehwerkzeug (12) ferner aufweist:
(a) eine auf dem länglichen Element gleitend aufgenommene Buchse (28), deren Länge geringer ist als diejenige des Elements; und
(b) eine auf dem länglichen Element angeordnete Markierungseinrichtung (60), durch die die Position der Buchse (28) bezüglich dem Element erfaßt werden kann, wobei die Markierungseinrichtung (60) einen auf dem länglichen Element (12) angeordneten Anschlag (60) aufweist, der so angeordnet ist, daß er mit der Buchse (28) in Kontakt kommen kann.

2. Vorrichtung nach Anspruch 1, wobei das längliche Element (12) aufweist:
(a) eine erste stabile zylinderförmige Stange mit einem proximalen und einem distalen Ende und mit einer Außenfläche, die durch ein um die Stange ausgebildetes spiralförmiges Gewinde gebildet wird; und
(b) eine am distalen Ende der ersten Stange befestigte zweite stabile zylinderförmige Stange, deren Durchmesser geringer ist als der Durchmesser der ersten Stange, wobei die zweite Stange sich von der ersten Stange ausgehend in axialer Richtung erstreckt.

3. Vorrichtung nach Anspruch 2, wobei das Ziehwerkzeug ferner eine am proximalen Abschnitt angeordnete Einrichtung (106) zum Befestigen eines Schlaghammers an der ersten Stange aufweist.

4. Vorrichtung nach Anspruch 2 oder 3, wobei die Außenfläche der zweiten Stange durch ein um die Stange ausgebildetes zweites spiralförmiges Gewinde gebildet wird.

5. Vorrichtung nach einem der vorangehenden Ansprüche, ferner mit:
(a) einem Formwerkzeug (10) zum Formen eines länglichen Gewindekanals in einer in den Hohlraum eingespritzten Zementmasse;
(b) einem zweiten länglichen Element (14) mit einer vorgegebenen Länge, die größer ist als diejenige des ersten Elements (12), wobei das zweite Element (14) einen Gewindeabschnitt (38) aufweist, der bezüglich dem durch das Formwerkzeug gebildeten Gewindekanal komplementär ausgebildet ist;
(c) einer auf dem zweiten länglichen Element (14) gleitend aufgenommenen zweiten Buchse (30), deren Länge geringer ist als diejenige des zweiten Elements (14);
(d) einer auf dem zweiten länglichen Element (14) angeordneten Markierungseinrichtung (62), durch die die Position der zweiten Buchse (30) bezüglich dem zweiten Element erfaßt werden kann;
(e) einer auf dem ersten und dem zweiten Element angeordneten Drehmomentübertragungseinrichtung (52, 54), durch die die Elemente in den durch das Formwerkzeug in der Zementmasse ausgebildeten Gewindekanal geschraubt in Eingriff gebracht werden können; und
(f) einer auf dem ersten und dem zweiten Element angeordneten Greifvorrichtung (106), durch die Zugkräfte auf die Elemente ausgeübt werden können, um eine Zementmasse, die mit den Gewindeabschnitten (36, 38) der Elemente (12, 14) in Gewindeeingriff gebracht wurde, aus einer Knochenvertiefung zu entfernen, wobei das erste längliche Element (12) einen Gewindeabschnitt (36) aufweist, der bezüglich dem durch das Formwerkzeug (10) gebildeten Gewindekanal komplementär ausgebildet ist.

6. Vorrichtung nach Anspruch 5, ferner mit einer Meßeinrichtung (20) zum Messen der Länge des Gewindekanals.

7. Vorrichtung nach Anspruch 6, wobei die Meßeinrichtung (20) ferner aufweist:
(a) ein längliches Stabelement (68) mit einem Durchmesser, der geringer ist als derjenige des Gewindekanals, und einer Länge, die größer ist als diejenige des Gewindekanals;
(b) ein um das Stabelement gleitend angeordnetes Rohr (74) mit einem distalen Abschnitt mit einem Querschnitt, der größer ist als der Durchmesser des Gewindekanals; und
(c) eine mit dem Stabelement (68) und dem Rohr (74) betrieblich verbundene Anzeigeeinrichtung (85) zum Anzeigen der Längsposition des Rohrs bezüglich des Stabelements.

8. Vorrichtung nach einem der vorangehenden Ansprüche, wobei die Markierungseinrichtung einen am länglichen Element befestigten Anschlag aufweist, der mit der Buchse in Kontakt kommen kann.

## Patentansprüche (Patentansprüche für folgende(n) Vertragsstaat(en): ES)

1. Vorrichtung zum Entfernen eines in einem haftenden Zustand in einer Knochenvertiefung angeordneten Zementmantels mit einem darin ausgebildeten länglichen Hohlraum, wobei die Vorrichtung ein Ziehwerkzeug (12) aufweist, das aus einem länglichen Element (12) mit einem Gewindeabschnitt gebildet wird, **dadurch gekennzeichnet, daß** das Ziehwerkzeug (12) ferner aufweist:
(a) eine auf dem länglichen Element gleitend aufgenommene Buchse (28), deren Länge geringer ist als diejenige des Elements; und
(b) eine auf dem länglichen Element angeordnete Markierungseinrichtung (60), durch die die Position der Buchse (28) bezüglich dem Element erfaßt werden kann.

2. Vorrichtung nach Anspruch 1, **dadurch gekennzeichnet, daß** die Markierungseinrichtung (60) einen auf dem länglichen Element angeordneten Anschlag (60) aufweist, der mit der Buchse (28) in Kontakt kommen kann.

3. Vorrichtung nach Anspruch 1 oder 2, **dadurch gekennzeichnet, daß** das längliche Element (12) aufweist:
(a) eine erste stabile zylinderförmige Stange mit einem proximalen und einem distalen Ende und mit einer Außenfläche, die durch ein um die Stange ausgebildetes spiralförmiges Gewinde gebildet wird; und
(b) eine am distalen Ende der ersten Stange befestigte zweite stabile zylinderförmige Stange, deren Durchmesser geringer ist als der Durchmesser der ersten Stange, wobei die zweite Stange sich von der ersten Stange ausgehend in axialer Richtung erstreckt.

4. Vorrichtung nach Anspruch 3, **dadurch gekennzeichnet, daß** das Ziehwerkzeug ferner eine am proximalen Abschnitt angeordnete Einrichtung (106) zum Befestigen eines Schlaghammers an der ersten Stange aufweist.

5. Vorrichtung nach Anspruch 3 oder 4, **dadurch gekennzeichnet, daß** die Außenfläche der zweiten Stange durch ein um die Stange ausgebildetes zweites spiralförmiges Gewinde gebildet wird.

6. Vorrichtung nach einem der vorangehenden Ansprüche, **dadurch gekennzeichnet, daß** die Vorrichtung ferner aufweist:
(a) ein Formwerkzeug (10) zum Formen eines länglichen Gewindekanals in einer in den Hohlraum eingespritzten Zementmasse;
(b) ein zweites längliches Element (14) mit einer vorgegebenen Länge, die größer ist als diejenige des ersten Elements (12), wobei das zweite Element (14) einen Gewindeabschnitt (38) aufweist, der bezüglich dem durch das Formwerkzeug gebildeten Gewindekanal komplementär ausgebildet ist;
(c) eine auf dem zweiten länglichen Element (14) gleitend aufgenommene zweite Buchse (30), deren Länge geringer ist als diejenige des zweiten Elements (14);
(d) eine auf dem zweiten länglichen Element (14) angeordnete Markierungseinrichtung (62), durch die die Position der zweiten Buchse (30) bezüglich dem zweiten Element erfaßt werden kann;
(e) eine auf dem ersten und dem zweiten Element angeordnete Drehmomentübertragungseinrichtung (52, 54), durch die die Elemente in den durch das Formwerkzeug in der Zementmasse ausgebildeten Gewindekanal geschraubt in Eingriff gebracht werden können; und
(f) eine auf dem ersten und dem zweiten Element angeordnete Greifvorrichtung (106), durch die Zugkräfte auf die Elemente ausgeübt werden können, um eine Zementmasse, die mit den Gewindeabschnitten (36, 38) der Elemente (12, 14) in Gewindeeingriff gebracht wurde, aus einer Knochenvertiefung zu entfernen, wobei das erste längliche Element (12) einen Gewindeabschnitt (36) aufweist, der bezüglich dem durch das Formwerkzeug (10) gebildeten Gewindekanal komplementär ausgebildet ist.

7. Vorrichtung nach Anspruch 6, **dadurch gekennzeichnet, daß** die Vorrichtung ferner eine Meßeinrichtung (20) zum Messen der Länge des Gewindekanals aufweist.

8. Vorrichtung nach Anspruch 7, **dadurch gekennzeichnet, daß** die Meßeinrichtung (20) ferner aufweist:
(a) ein längliches Stabelement (68) mit einem Durchmesser, der geringer ist als derjenige des Gewindekanals, und einer Länge, die größer ist als diejenige des Gewindekanals;
(b) ein um das Stabelement gleitend angeordnetes Rohr (74) mit einem distalen Abschnitt mit einem Querschnitt, der größer ist als der Durchmesser des Gewindekanals; und
(c) eine mit dem Stabelement (68) und dem Rohr (74) betrieblich verbundene Anzeigeeinrichtung (85) zum Anzeigen der Längsposition des Rohrs bezüglich dem Stabelement.

9. Vorrichtung nach einem der vorangehenden Ansprüche, wobei die Markierungseinrichtung einen am länglichen Element befestigten Anschlag aufweist, der mit der Buchse in Kontakt kommen kann.

## Revendications (Revendications pour l'(les) Etat(s) contractant(s) suivant(s): AT, BE, CH, DE, FR, GB, IT, LI, NL, SE)

1. Ensemble pour enlever une enveloppe en ciment ayant une cavité oblongue formée dans celle-ci d'un état d'adhésion dans un évidement d'os, ledit ensemble comprenant un outil de traction (12) constitué d'un élément oblong (12) ayant une section à filetage de vis, ledit outil de traction (12) comprenant en outre ;
(a) un manchon (28) reçu de façon coulissante sur l'élément oblong, ledit manchon (28) ayant une longueur inférieure à celle de l'élément ; et
(b) un moyen de marquage (60) sur l'élément oblong pour permettre l'établissement d'une comparaison de la position du manchon (28) relativement à l'élément, ledit moyen de marquage (60) comprenant une butée d'arrêt (60) sur l'élément oblong (12) disposé pour buter contre le manchon (28).

2. Ensemble selon l'une des revendications précédentes, dans lequel l'élément oblong (12) comprend :
(a) un premier montant cylindrique rigide ayant des extrémités proximale et distale et possédant une surface extérieure d'un filetage de vis hélicoïdal réalisé autour dudit montant ; et
(b) un deuxième montant cylindrique rigide fixé à l'extrémité distale dudit premier montant, ledit deuxième montant ayant un diamètre réduit comparé à celui dudit premier montant et s'étendant axialement de celui-ci.

3. Ensemble selon la revendication 2, dans lequel l'outil de traction comprend en outre un moyen (106) sur ladite section proximale pour fixer un marteau de frappe audit premier montant.

4. Ensemble selon la revendication 2 ou la revendication 3, dans lequel le deuxième montant a une surface extérieure présentant un deuxième filetage de vis hélicoïdal formé autour dudit deuxième montant.

5. Ensemble selon l'une des revendications précédentes, dans lequel l'ensemble comprend en outre :
(a) un taraud (10) pour former un passage oblong à filetage de vis dans une masse de ciment injectée dans la cavité ;
(b) un deuxième élément oblong (14) d'une longueur prédéterminée qui est plus grande que celle du premier élément (12), ledit deuxième élément (14) ayant une section à filetage de vis (38) complémentaire au passage à filetage de vis réalisé par le taraud ;
(c) un deuxième manchon (30) reçu de façon coulissante sur le deuxième élément oblong (14), ledit manchon ayant une longueur inférieure à celle du deuxième élément (14) ; et
(d) un moyen de marquage (62) sur le deuxième élément oblong (14) pour permettre l'établissement d'une comparaison de la position du deuxième manchon (30) relativement au deuxième élément ;
(e) un moyen de transmission de couple (52, 54) sur lesdits premier et deuxième éléments pour permettre que lesdits éléments soient amenés en prise de filetage avec un passage fileté ménagé dans une masse de ciment par le taraud ; et
(f) un moyen de préhension (106) sur lesdits premier et deuxième éléments pour permettre l'application de forces de traction à ceux-ci afin d'enlever une masse de ciment en prise de filetage avec les sections à filetage de vis (36, 38) des éléments (12, 14) d'un évidement d'os : et en ce que le premier élément oblong (12) a une section de filetage de vis (36) complémentaire avec un passage à filetage de vis réalisé par le taraud (10).

6. Ensemble selon la revendication 5, dans lequel l'ensemble comporte en outre une jauge (20) pour mesurer la longueur du passage à filetage de vis.

7. Ensemble selon la revendication 6, dans lequel la jauge (20) comprend :
(a) une tige allongée (68) ayant un diamètre inférieur à celui du passage à filetage de vis et une longueur plus grande que celle dudit passage ;
(b) un tube (74) disposé de façon coulissante autour de la tige, ledit tube ayant une portion distale d'une section transversale qui est plus grande que le diamètre du passage fileté ; et
(c) un moyen d'indice (85) opérationnellement associé à la tige (68) et au tube (74) pour indiquer la position longitudinale du tube relativement à la tige.

8. Ensemble selon l'une des revendications précédentes, dans lequel le moyen de marquage comprend un collier fixé à l'élément oblong pour buter contre le manchon.

## Revendications (Revendications pour l'(les) Etat(s) contractant(s) suivant(s): ES)

1. Ensemble pour enlever une enveloppe en ciment présentant une cavité oblongue formée dans celle-ci d'un état d'adhésion dans un évidement d'os, ledit ensemble comprenant un outil de traction (12) constitué d'un élément oblong (12) ayant une section à filetage de vis, caractérisé en ce que l'outil de traction (12) comprend en outre :
(a) un manchon (28) reçu de façon coulissante sur l'élément oblong, ledit manchon (28) ayant une longueur inférieure à celle de l'élément, et
(b) un moyen de marquage (60) sur l'élément oblong pour permettre l'établissement d'une comparaison de la position du manchon (28) relativement à l'élément.

2. Ensemble selon l'une des revendications précédentes, caractérisé en ce que le moyen de marquage (60) comprend une butée d'arrêt (60) sur l'élément oblong disposée pour buter contre le manchon (28).

3. Ensemble selon l'une des revendications précédentes, caractérisé en ce que l'élément oblong (12) comprend :
(a) un premier montant cylindrique rigide ayant des extrémités proximale et distale, et présentant une surface extérieure d'un filetage de vis hélicoïdal formé autour dudit montant, et
(b) un deuxième montant cylindrique rigide fixé à l'extrémité distale dudit premier montant, ledit deuxième montant ayant un diamètre réduit comparé audit premier montant et s'étendant axialement de celui-ci.

4. Ensemble selon la revendication 3, caractérisé en ce que l'outil de traction comprend en outre un moyen (106) sur ladite section proximale pour fixer un marteau de frappe audit premier montant.

5. Ensemble selon la revendication 3 ou la revendication 4, caractérisé en ce que le deuxième montant a une surface extérieure d'un deuxième filetage de vis hélicoïdal formé autour dudit deuxième montant.

6. Ensemble selon l'une des revendications précédentes, caractérisé en ce que l'ensemble comprend en outre:
(a) un taraud (10) pour réaliser un passage oblong à filetage de vis dans une masse de ciment injectée dans la cavité ;
(b) un deuxième élément oblong (14) d'une longueur prédéterminée qui est plus grande que celle du premier élément (12), ledit deuxième élément (14) ayant une section à filetage de vis (38) complémentaire à un passage à filetage de vis réalisé par le taraud ;
(c) un deuxième manchon (30) reçu de façon coulissante sur le deuxième élément oblong (14), ledit manchon ayant une longueur inférieure à celle du deuxième élément (14) ; et
(d) un moyen de marquage (62) sur le deuxième élément oblong (14) pour permettre l'établissement d'une comparaison de la position du deuxième manchon (30) relativement au deuxième élément ;
(e) un moyen de transmission de couple (52, 54) sur lesdits premier et deuxième éléments pour permettre que lesdits éléments soient amenés en prise de filetage avec un passage fileté réalisé dans une masse de ciment par le taraud ; et
(f) un moyen de préhension (106) sur lesdits premier et deuxième éléments pour permettre l'application de forces de traction à ceux-ci afin d'enlever une masse de ciment en prise de filetage avec les sections à filetage de vis (36, 38) des éléments (12, 14) d'un évidement d'os ; et en ce que le premier élément oblong (12) a une section à filetage de vis (36) complémentaire avec un passage à filetage de vis réalisé par le taraud (10).

7. Ensemble selon la revendication 6, caractérisé en ce que l'ensemble comporte en outre une jauge (20) pour mesurer la longueur du passage à filetage de vis.

8. Ensemble selon la revendication 7, caractérisé en ce que la jauge (20) comprend :
(a) une tige allongée (68) ayant un diamètre inférieur à celui du passage à filetage de vis et une longueur plus grande que celle dudit passage ;
(b) un tube (74) disposé de façon coulissante autour de la tige, ledit tube ayant une portion distale d'une section transversale plus grande que le diamètre du passage fileté; et
(c) un moyen d'indice (85) associé opérationnellement à la tige (68) et au tube (74) pour indiquer la position longitudinale du tube relativement à la tige.

9. Ensemble selon l'une des revendications précédentes, dans lequel le moyen de marquage comprend un collier fixé à l'élément oblong pour buter contre le manchon.
